# EUROPEAN PATENT APPLICATION

(11) **EP 4 599 875 A1**
(43) Date of publication of application: **13.08.2025**
(21) Application number: 23875224.0
(22) Date of filing: 05.10.2023
(51) Int. Cl.: A61M 16/04, A61M 16/00, A61B 1/267, A61B 1/00, A61B 1/05

(54) **ENDOTRACHEAL INTUBATION GUIDE DEVICE AND ENDOTRACHEAL INTUBATION GUIDE SYSTEM**

(30) Priority: 06.10.2022 KR 20220127767
(71) Applicant: Seoul National University Hospital, Seoul 03080 (KR)
(72) Inventor: KIM, Dae Kon, Seongnam-si, Gyeonggi-do 13599 (KR)
(74) Representative: V.O.
(86) International application number: PCT/KR2023/015295
(87) International publication number: WO 2024/076162

(57) **Abstract**

An endotracheal intubation guide device according to an embodiment of the present disclosure may comprise: an insertion case which is bent along a lengthwise direction to be inserted into the mouth and has an opening formed through an upper part thereof; a balloon provided at the insertion case to widen the mouth; and a tube which is connected to the balloon to inject air into the balloon and thus expand the balloon and is connected to a front part of the balloon, wherein the balloon may be disposed at the opening of the insertion case to enable the balloon to come into contact with a ceiling of the mouth when air is injected through the tube.

## Description

### Technical Field

The present disclosure relates to an endotracheal intubation guide device and an endotracheal intubation guide system. Specifically, the present disclosure relates to an endotracheal intubation guide device and an endotracheal intubation guide system capable of easily intubating an E-tube into a trachea.

### Background Art

Out-of-hospital cardiac arrest (OHCA) is an important problem in public health issue, occurring in, for example, 420,000 people per year in the United States, with a survival rate of only about 10.4%. In the case of OHCA, paramedics arrive after the patient's report and perform cardiopulmonary resuscitation (CPR), where securing the trachea is critical, and for this purpose endotracheal intubation (ETI) is particularly important.

However, despite its importance, ETI is difficult to learn, requires a lot of experience in learning the skills, and there is a concern that it may cause harm to patients in the event of failing to perform ETI. In addition, a CPR delay for ETI may result in a rather poor prognosis of the patient.

Accordingly, paramedics and the like who have little experience in ETI tend to prefer the insertion of a supraglottic airway device (SAD) rather than ETI on the emergency scene.

However, in the SAD insertion method, the SAD is easy to insert, but it often does not properly enter the trachea, due to dislodgement or esophagus insertion and the like, and thus the trachea cannot be efficiently secured. In addition, although ETI is difficult, ETI is a gold-standard technique for securing the trachea, so attempting ETI is helpful for the prognosis of the patient.

Conventionally, there is a guide device for ETI developed by the present inventor (e.g., Patent Document 1), and this device is easy to guide ETI through a handle or the like that lifts the guide device in a direction opposite to gravity. However, according to the present inventor's understanding, even when this device is used, the mouth of a patient still needs to be widened to a greater extent, and thus an easier ETI technique is needed.

### Prior Art Document

### Patent Document

Patent Document 1: Korean Patent Application No. 10-2020-0096290

### Detailed Description of the Invention

### Technical Problem

According to an aspect of the present disclosure, there is provided an endotracheal intubation (ETI) guide device which, in endotracheal tube (E-tube) insertion, makes it easier to perform ETI into the trachea and not into the esophagus by simply inflating the mouth and inserting the E-tube even in a blind state in which the trachea is not visible, so that ETI may be easily performed even by an operator such as unskilled paramedics.

In another aspect, the present disclosure provides an ETI guide device that may be easily combined with a video laryngoscope and may be effectively used as a video laryngoscope device. In particular, when the mouth is widened through a balloon, the range that may be photographed through the video laryngoscope is widened, so that ETI may be more easily performed when using the video laryngoscope, and therefore, the present disclosure provides an ETI guide device and an ETI guide system capable of adjusting the degree of air injection by the balloon in response to the mouth image photographed with the video laryngoscope, and thus appropriately widening the mouth.

### Solution to Problem

An endotracheal intubation (ETI) guide device according to an embodiment of the present disclosure may include: an insertion case which is bent along a lengthwise direction to be inserted into the mouth and has an opening formed through an upper part thereof; a balloon provided at the insertion case to widen the mouth; and a tube which is connected to the balloon to inject air into the balloon and thus expand the balloon and is connected to a front part of the balloon, wherein the balloon may be disposed at the opening of the insertion case to enable the balloon to come into contact with a ceiling of the mouth when air is injected through the tube.

One end of the insertion case may be provided with a tube insertion unit opened so that the tube is inserted into a body, wherein the other end of the insertion case faces the inside of the trachea.

The ETI guide device may further include: an epiglottis hanging portion provided on a lower side of the other end of the insertion case when the insertion case is inserted into the trachea; and an esophagus insertion prevention tube provided on an upper side of the other end of the insertion case when the insertion case is inserted into the trachea and protruding farther in a lengthwise direction of the insertion case than the epiglottis hanging portion.

The balloon may be disposed between one end and the other end of the insertion case.

The ETI guide device may further include a video laryngoscope configured to observe the trachea, the video laryngoscope including: a camera disposed on the other end of the insertion case and configured to photograph the trachea; and a display disposed on one end of the insertion case and connected to the camera to display information obtained from the camera.

An ETI guide system according to the present disclosure may further include the ETI guide device, and an air injection device for injecting air into a tube of a balloon of the ETI guide device, wherein the air injection device includes an air injection amount control unit, wherein the air injection amount control unit is provided with a processor, and the processor performs the steps of receiving a mouth photographed image obtained through a video laryngoscope, determining a volume of the mouth from the image, and determining air injection amount based on the determined volume.

### Effects of the Invention

According to an aspect of the present disclosure, since the mouth may be widened, it is possible to intubate the E-tube into the trachea even in a blind state, reducing the possibility of erroneous insertion into the esophagus, and secure more easily and certainly the trachea.

In addition, in particular, when the mouth is widened through the balloon, a range in which the mouth may be photographed through the video laryngoscope is widened, so that the endotracheal intubation (ETI) may be more easily performed when the video laryngoscope is used, and furthermore, the degree of air injection by the balloon may be adjusted in response to the mouth image photographed by the video laryngoscope scope, so that the mouth is appropriately widened.

Accordingly, it is possible to lower the entry barrier of attempting ETI of an operator and facilitate ETI attempt in situations in which it is important to secure trachea such as in a first aid site, an emergency room, an operating room, and an intensive care unit. In particular, trachea securing techniques in the prehospital stage may be made possible.

### Brief Description of the Drawings

FIG. 1 shows a perspective view illustrating an endotracheal intubation (ETI) guide device according to an embodiment of the present disclosure.
FIG. 2 shows an exploded perspective view of the ETI guide device shown in FIG. 1.
FIG. 3 shows a schematic view illustrating a state before the ETI guide device shown in FIG. 1 is provided to a subject.
FIG. 4 shows a schematic view illustrating a state in which the ETI guide device shown in FIG. 1 is provided into a mouth of the subject.
FIG. 5 shows a schematic view illustrating the provision of an E-tube through an ETI guide passage after the ETI guide device shown in FIG. 1 has been provided into the mouth of the subject.
FIG. 6 shows a schematic side view of the ETI guide device shown in FIG. 1 after it is provided into the mouth of the subject.
FIG. 7 shows a control block diagram of the ETI guide device and an ETI guide system according to the present disclosure.

### Best Mode for Carrying Out the Invention

Hereinafter, embodiments of the present disclosure will be described with reference to the drawings. The present disclosure is described with reference to the embodiments shown in the drawings, but the embodiments are illustrative, and the technical concept of the present disclosure and the essential components and operations thereof are not limited thereby.

Top and bottom or upper and lower or right and left are to be understood herein as relative positional concepts. For example, it will be understood that left and right may be referred to as right and left depending on the viewing direction.

Being able to be assembled and disassembled herein means that not only each component of an endotracheal intubation (ETI) guide device may be assembled to use the device, but also the device may be disassembled and used in the mouth after ETI. To this end, in the ETI device of the exemplary embodiments of the present disclosure, individual components are fastened so as to be easily separated and coupled.

Terms such as "unit," "module" or "system" as used herein may refer to hardware as well as a combination with software driven by the hardware. For example, the hardware may be a data processing device including a Central Processing Unit (CPU), a Graphic Processing Unit (GPU), or another processor. Software may also refer to a process running, an object, an executable, a thread of execution, a program, and the like.

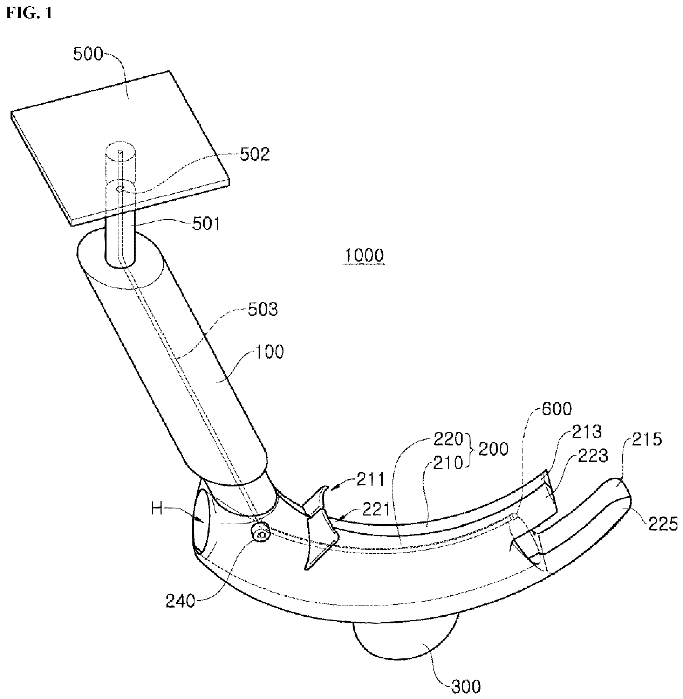

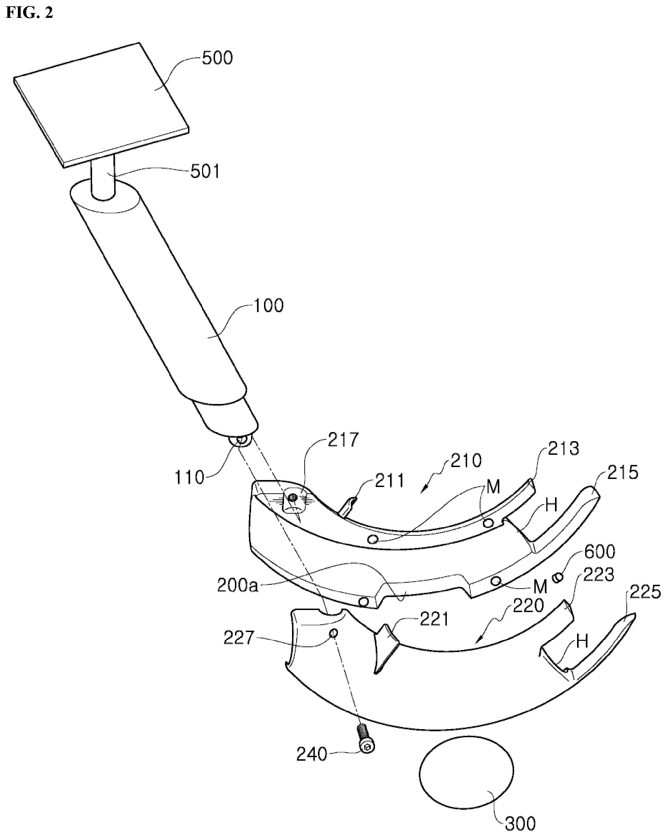

FIG. 1 shows a perspective view illustrating an ETI guide device according to an embodiment of the present disclosure. FIG. 2 shows an exploded perspective view of the ETI guide device shown in FIG. 1.

Referring to FIGS. 1 and 2, the ETI guide device 1000 according to an embodiment includes a cylindrical insertion case 200 that is bent along a lengthwise direction to be be inserted into the mouth.

As shown in FIG. 1 and FIG. 2 described below, the insertion case 200 may be bent such that it forms a descending curve from a proximal side to a central portion of the insertion case 200 and forms an ascending curve again from the central portion to the proximal side of the case 200 so as to be suitable for entering a trachea after intraoral insertion.

In the insertion case 200, a first case body 210 constituting a right side half of the case (right side when viewed from the distal side of the case) and a second case body 220 constituting a left side half of the case (left side when viewed from the distal side of the case) may be coupled by one or more magnets M. Here, the first case body 210 and the second case body 220 may be symmetrical.

In detail, in the ETI guide device 1000 of the embodiments of the present disclosure, individual components must be fastened to enable separation and coupling.

First, the insertion case 200 of the ETI guide device is fastened such that the first case body 210 constituting the right half of the case and the second case body 220 constituting the left half of the case may be separated and coupled to each other to constitute the insertion case 200.

In one embodiment, the first case body 210 and the second case body 220 may be fastened by a fastening means that facilitates separation and coupling, such as, , one or more magnets M described above, but are not limited thereto. For example, a protruding portion may be formed on any one side of coupling surfaces of the first case body 210 and the second case body 220, and a receiving portion for receiving the protruding portion may be formed in the other side, so that the protruding portion is fastened and separated such that it is fitted into and removed from the receiving portion.

The case formed by coupling the first case body 210 and the second case body 220 is provided with an endotracheal tube insertion hole on a proximal side of the case, an endotracheal tube discharge hole on the proximal side of the case, and an ETI guide passage H connecting the endotracheal tube insertion hole and the endotracheal tube discharge hole and curved in a lengthwise direction.

One side of a distal end portion of the insertion case 200 is provided with an epiglottis hanging portion 213 and 223 protruding from the case. When the ETI guide device according to an embodiment enters along the tongue, the epiglottis hanging portion 213 and 223 is located between the epiglottis and a base of the tongue. Hanging a tooth hanging portion 211 and 221 between two teeth in the middle of the mandible of the subject 0 enables the ETI guide device to be placed in a correct position in the mouth. Then, when the ETI guide device is lifted in the direction of the sky, the epiglottis hanging portion 213 and 223 that is hung over the epiglottis is pushed up due to the anatomy and serves to push (open) the epiglottis to expose the trachea.

In addition, the other side of the distal end portion of the case is provided with an esophagus insertion prevention plate 215 and 225 that protrudes farther from the case 200 than the epiglottis hanging portion 213 and 223 in order to prevent esophagus insertion.

A discharge hole of the ETI guide passage H is located in a region between the epiglottis hanging portion 213 and 223 and the esophagus insertion prevention plate 215 and 225. As a result, the E-tube that has passed through the guide passage H easily enters the trachea in a state in which the epiglottis is pushed up and opened by the epiglottis hanging portion 213 and 223 and in a state of being blocked from entering the esophagus by the esophagus insertion prevention plate 215 and 225.

The ETI guide device may be further provided with a tooth hanging portion 211 and 221 on one side of the proximal side of the case.

The tooth hanging portion 211 and 221 spans between the two teeth in the middle of the mandible of the subject 0 when the ETI guide device is provided into the mouth of the subject 0. Such hanging of the teeth with the tooth hanging portion 211 and 221 may help in positioning a correct starting position of the ETI guide device. Accordingly, since the endotracheal tube E only needs to be pushed in, ETI may be easily performed.

The ETI guide device may further include a handle 100 coupled to the case.

As described above, the ETI guide device of the embodiments of the present disclosure requires individual components to be fastened to enable separation and coupling. Therefore, not only the above-mentioned first case body 210 and the above-mentioned second case body 220 need to be easily separated and coupled, but also the handle 100 needs to be easily separated and coupled.

To this end, the handle 100, the first case body 210 and the second case body 220 of the ETI guide device may be fastened so as to be separated from and coupled with each other by one fastening means at a handle coupling unit.

For example, in one embodiment, the handle 100 may include a screw through hole 110, and the screw through hole 110 may be coupled to the first case body 210 and the second case body 220 by using a screw 240 at the handle coupling unit 217 and 227.

Here, the handle 100 and the handle coupling unit 217 and 227 are located on the rear side of the tooth hanging portion 211 and 221 on the proximal side of the case.

The ETI guide device 1000 according to an embodiment of the present disclosure further includes an opening 200a and a balloon 300.

The opening 200a is formed at the insertion case 200. The opening 200a is disposed on an upper part thereof when the insertion case 200 is inserted into the mouth. The opening 200a may be provided on a central portion side of the insertion case 200. However, the position of the opening 200a is not limited to that shown in the drawings. The balloon 300 is disposed at the opening 200a.

The balloon 300 is provided in the insertion case 200 to widen the mouth. The balloon 300 may be fitted into the insertion case 200 through the opening 200a, or attached to the insertion case 200. The balloon 300 may be inflated in a manner similar to a Foley catheter. For example, the balloon 300 may be inflated or deflated by a tube 400 described below. The balloon 300 may be formed of a rubber material.

The ETI guide device 1000 according to an embodiment further includes a video laryngoscope 700, a connection member 501, and a hinge 502. The video laryngoscope 700 includes a display 500 and a camera 600. The camera 600 and the display 500 are electrically connected.

The camera 600 may be disposed on one side of the insertion case 210 and 220. For example, the camera 600 may be disposed on a side of the epiglottis hanging portion 213 and 223. The position of the camera 600 is not limited to the above-described example. The camera 600 may photograph the epiglottis and the trachea and communicate the obtained information to the display 500.

The display 500 may be disposed on a side of the handle 100. The display 500 may be connected to the camera 600 through the connection member 501 connected to the handle and a wire 503 passing through the handle 100. The wire 503 may be attached to an inner wall of the insertion case 210 and 220 forming the guide passage H. However, the position of the wire 503 is not limited to the above-described example, and a separate wire passage (not shown) may be formed.

The display 500 and the connection member 501 are rotatably coupled to each other through the hinge 502. The hinge 502 enables the display 500 to rotate at an angle that enables a user to easily view the display 500.

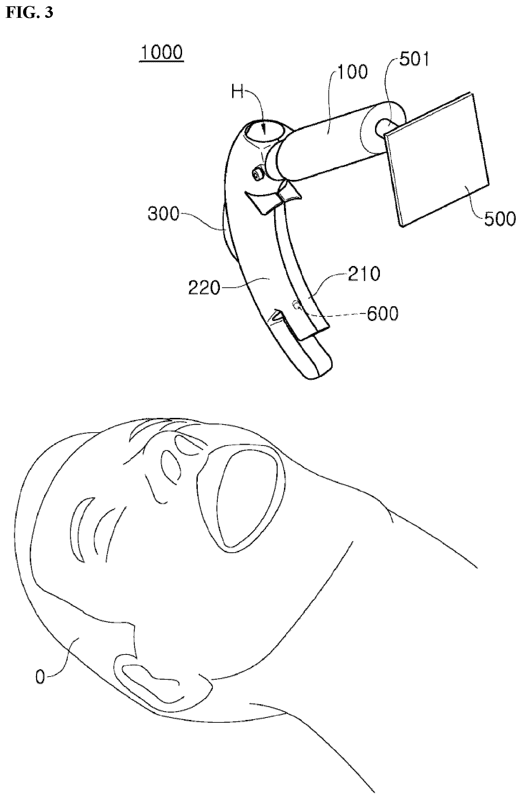

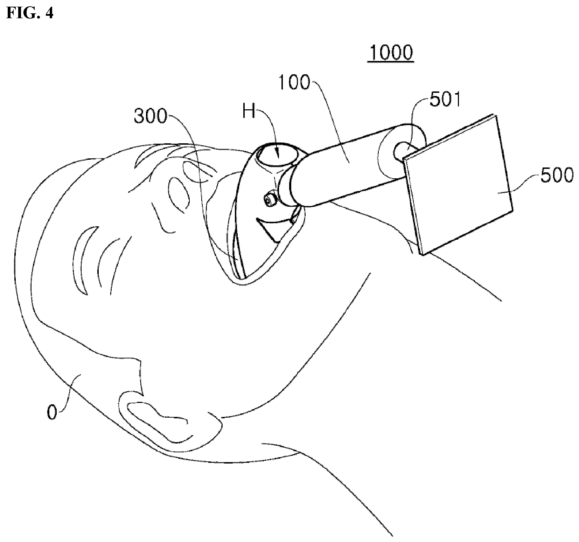

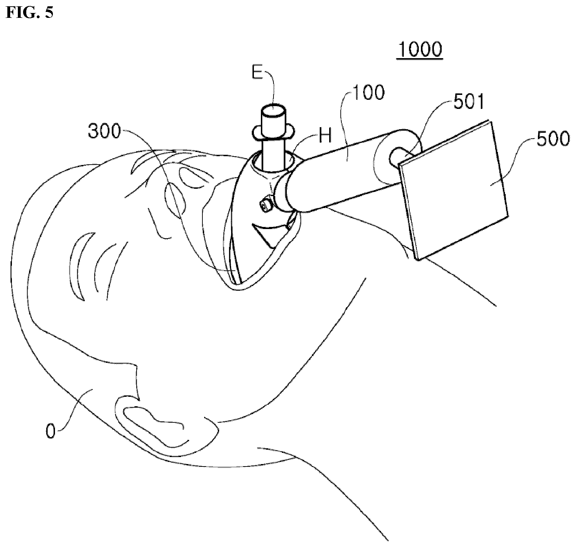

FIG. 3 shows a schematic view illustrating a state before the ETI guide device shown in FIG. 1 is provided to a subject. FIG. 4 shows a schematic view illustrating a state in which the ETI guide device shown in FIG. 1 is provided into a mouth of the subject. FIG. 5 shows a schematic view illustrating the provision of an E-tube through an ETI guide passage after the ETI guide device shown in FIG. 1 has been provided into the mouth of the subject.

As shown in FIGS. 3 to 5, the E-tube is provided through the guide passage H after the ETI guide device 1000 is provided to the subject 0.

As described above, the device of the present disclosure includes the epiglottis hanging portion 213 and 223 protruding from the distal end portion of the case and the esophagus insertion prevention plate 215 and 225 protruding farther from the case than the epiglottis hanging portion 213 and 223, and a tract is formed in advance in a direction of the trachea while blocking a direction of the esophagus.

As a result, the structure enables a tip of the E-tube to enter the trachea without entering the esophagus. Therefore, in this state, the operator may insert the E-tube into the trachea even in a blind state by simply pushing the E-tube through the guide passage H. Accordingly, it is possible to lower the entry barrier of attempting ETI of an operator and facilitate ETI attempt of an ETI operator such as paramedics, and the operator may comfortably use the E-tube even in a hospital where it is very important to secure the trachea such as in an emergency room, an operating room, and the like.

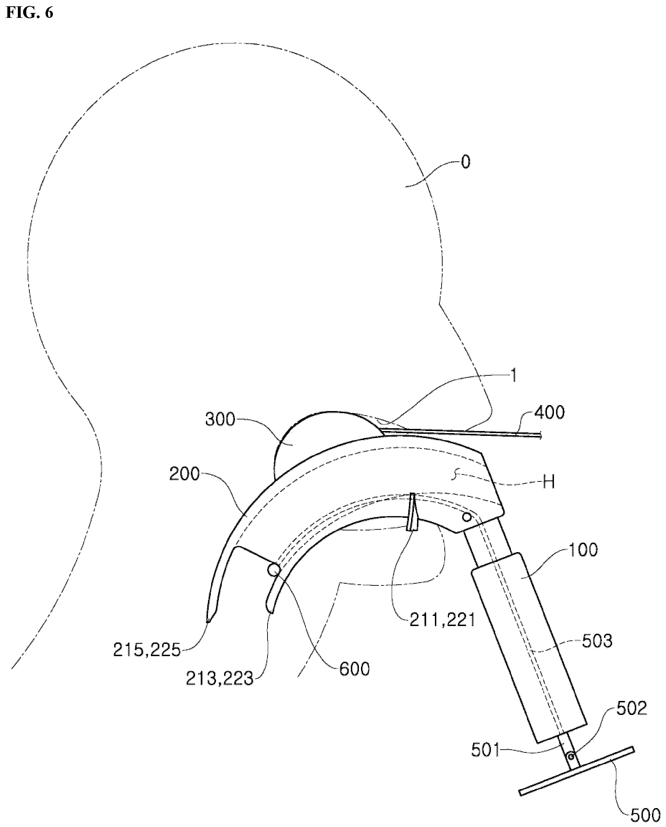

FIG. 6 shows a schematic side view of the ETI guide device shown in FIG. 1 after it is provided into the mouth of the subject.

Referring to FIG. 6, the ETI guide device 1000 according to an embodiment of the present disclosure may be inserted into the mouth to guide ETI.

When the insertion case 200 is disposed in the mouth, air may be injected from the outside into the balloon 300 through the tube. A plurality of tubes may be provided. The balloon 300 may expand as air is injected. The balloon 300 is disposed at the opening 200a, and the opening 200a is disposed on an upper part of the insertion case 200 when the insertion case is inserted into the mouth. Thus, the balloon 300 may be brought into contact with a ceiling 1 of the mouth as the balloon 300 expands. As the balloon 300 gradually expands, the insertion case 200 is pressed downward, and the insertion case 200 may move toward the trachea. **In** other words, the balloon 300 may be inflated to lift the epiglottis to expose the vocal cords and insert the endotracheal tube E as shown in FIG. 5. After the ETI is confirmed, air is removed from the balloon 300 to shrink the balloon, and the insertion case 200 is separated from the mouth to fix the intubated tube E.

As the insertion case 200 approaches the side of the trachea, esophageal intubation may be prevented, and ETIs may be easily performed in the subject 0. Accordingly, it is possible to lower the entry barrier of attempting ETI of an operator and facilitate ETI attempt of an ETI operator such as paramedics, and the operator may comfortably use the E-tube even in a hospital where it is very important to secure the trachea such as in an emergency room, an operating room, and the like.

When the insertion case 200 is inserted into the trachea, the camera 600 may be disposed below the other end of the insertion case 210 and 220 with reference to the drawings. In addition, the display 500 may be disposed at the lowermost end of the handle 100 with reference to the drawing when the insertion case 210 and 220 is inserted into the trachea.

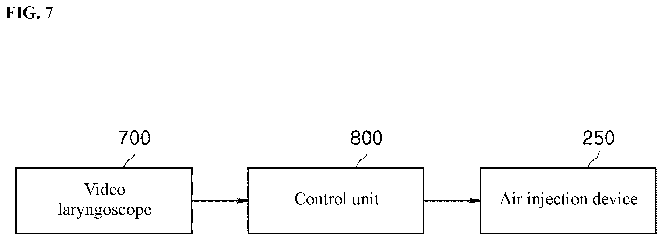

In addition, when the insertion case 200 is inserted into the mouth, the epiglottis hanging portion 213 and 223 and the esophagus insertion prevention plate 215 and 225 may prevent the tongue in the mouth from moving freely. At this time, the user may observe the epiglottis and the trachea using a video laryngoscope 700, or check the status of ETI. In addition, the hinge 502 coupled to the display 500 enables the adjustment to an angle that is convenient for the user. FIG. 7 shows a control block diagram of the ETI guide device and an ETI guide system according to the present disclosure.

Referring to FIG. 7, an air injection amount of the balloon 300 may be adjusted in response to a photographed mouth image obtained through the video laryngoscope 700 and an oral condition confirmed therefrom.

The ETI guide system according to the present disclosure includes an air injection device 250 connected to the balloon 300, and the air injection device 250 includes an air injection amount control unit 800. The control unit 800 may include a processor configured to receive a mouth image obtained through the video laryngoscope 700, determine a volume of the mouth from the image, and determine an air inj ection amount based on the determined volume. The processor may control the air injection device 250 by determining an air injection amount, and air may be injected into the balloon 300 via the air injection device 250. The control unit 800 may be included in the air injection device 250.

Accordingly, in one aspect, the ETI guide system according to the present disclosure includes the ETI guide device and an air injection device 250 that injects air into a tube 400 of a balloon of the ETI guide device, and the air injection device 250 includes an air injection amount control unit 800. The air injection amount control unit 800 may include a processor configured to receive a mouth image obtained through a video laryngoscope 700, determine a volume of the mouth from the image, and determine an air injection amount based on the determined volume. The processor may control the air injection device 250 by determining an air injection amount, and air may be injected into the balloon 300 via the air injection device 250. The control unit 800 may be included in the air injection device 250.

With reference to the foregoing descriptions, a person skilled in the art may understand that the present disclosure may be implemented in other specific forms without changing the technical concept or essential characteristics thereof.

Therefore, it should be understood that the above-described embodiments are illustrative in all respects, and are not intended to limit the present disclosure thereto, and the scope of the present disclosure is defined by the following claims rather than the detailed description above, and all changes or modified forms derived from the meaning and scope of the claims and equivalent concepts should be construed as being included in the scope of the present disclosure.

### Industrial Applicability

The endotracheal intubation (ETI) guide device and system according to the embodiments of the present disclosure may more easily and certainly secure the trachea.

## Claims

1. An endotracheal intubation guide device, comprising:
an insertion case which is bent along a lengthwise direction to be inserted into the mouth and has an opening formed through an upper part thereof;
a balloon provided at the insertion case to widen the mouth; and
a tube which is connected to the balloon to inject air into the balloon and thus expand the balloon and is connected to a front part of the balloon,
wherein the balloon may be disposed at the opening of the insertion case to enable the balloon to come into contact with a ceiling of the mouth when air is injected through the tube.

2. The device of claim 1, wherein one end of the insertion case is provided with a tube insertion unit opened so that the tube is inserted into the body, wherein the other end of the insertion case faces the inside of the trachea.

3. The device of claim 1, further comprising:
an epiglottis hanging portion provided on a lower side of the other end of the insertion case when the insertion case is inserted into the trachea; and
an esophagus insertion prevention tube provided on an upper side of the other end of the insertion case when the insertion case is inserted into the trachea and protruding farther in a lengthwise direction of the insertion case than the epiglottis hanging portion.

4. The device of claim 3, wherein the balloon is disposed between one end and the other end of the insertion case.

5. The device of claim 1, further comprising a video laryngoscope configured to observe the trachea, the video laryngoscope comprising:
a camera disposed on the other end of the insertion case and configured to photograph the trachea; and
a display disposed on one end of the insertion case and connected to the camera to display information obtained from the camera.

6. An endotracheal intubation guide system further comprising:
the endotracheal intubation guide device according to claim 5; and
an air injection device for injecting air into a tube of a balloon of the endotracheal intubation guide device,
wherein the air injection device comprises an air injection amount control unit,
wherein the air injection amount control unit is provided with a processor, and the processor performs the steps of:
receiving a mouth photographed image obtained through a video laryngoscope;
determining a volume of the mouth from the image; and
determining air injection amount based on the determined volume.
